# EUROPEAN PATENT SPECIFICATION

(11) **EP 3 185 874 B1**
(45) Date of publication and mention of the grant of the patent: **28.04.2021**
(21) Application number: 15762769.6
(22) Date of filing: 28.08.2015
(51) Int. Cl.: A61K 31/702, A61P 43/00

(54) **METHOD OF IMPROVING OR ENHANCING GROWTH AT AN ADULT AGE OF AN OFFSPRING BY ADMINISTRATION OF FOS**
VERFAHREN ZUR VERBESSERUNG ODER ERWEITERUNG DES WACHSTUMS IM ERWACHSENENALTER EINES NACHKOMMENS DURCH VERABREICHUNG VON FOS
PROCÉDÉ PERMETTANT D'AMÉLIORER OU DE STIMULER LA CROISSANCE À UN ÂGE ADULTE D'UNE PROGÉNITURE PAR ADMINISTRATION DE FOS

(30) Priority: 29.08.2014 EP 14182957
(43) Date of publication of application: 05.07.2017
(73) Proprietor: Beghin Meiji SA, 59777 Euralille (FR)
(72) Inventor: APPER, Emmanuelle, 82220 Labarthe (FR); RESPONDEK, Frédérique, F-67600 Ebersheim (FR)
(74) Representative: Icosa
(86) International application number: PCT/IB2015/056536
(87) International publication number: WO 2016/030861

(56) References cited:
- EP-A1- 2 653 040
- EP-A2- 0 133 547
- CN-A- 102 613 409
- CN-A- 103 636 977
- US-A1- 2013 189 398
- GRAND E ET AL: "Effects of short-chain fructooligosaccharides on growth performance of preruminant veal calves.", JOURNAL OF DAIRY SCIENCE FEB 2013, vol. 96, no. 2, February 2013 (2013-02), pages 1094-1101, XP002734533, ISSN: 1525-3198
- XU CHUANLAI ET AL: "Study of the application of fructooligosaccharides in piglets", ASIAN-AUSTRALASIAN JOURNAL OF ANIMAL SCIENCES, vol. 18, no. 7, July 2005 (2005-07), pages 1011-1016, XP002734534, ISSN: 1011-2367
- DATABASE BIOSIS [Online] BIOSCIENCES INFORMATION SERVICE, PHILADELPHIA, PA, US; April 2011 (2011-04), KIM EUN JIN: "Effects of Fructooligosaccharides Intake on Body Weight, Lipid Profiles, and Calcium Status among Korean College Students", XP002734535, Database accession no. PREV201300066604 & FASEB JOURNAL, vol. 25, April 2011 (2011-04), EXPERIMENTAL BIOLOGY MEETING 2011; WASHINGTON, DC, USA; APRIL 09 -13, 2011 ISSN: 0892-6638(print)
- FUJIWARA REIKO ET AL: "Maternal consumption of fructo-oligosaccharide diminishes the severity of skin inflammation in offspring of NC/Nga mice.", THE BRITISH JOURNAL OF NUTRITION FEB 2010, vol. 103, no. 4, February 2010 (2010-02), pages 530-538, XP002734536, ISSN: 1475-2662
- V. ADOGONY ET AL: "Effects of dietary scFOS on immunoglobulins in colostrums and milk of bitches", JOURNAL OF ANIMAL PHYSIOLOGY AND ANIMAL NUTRITION., vol. 91, no. 5-6, 16 May 2007 (2007-05-16) , pages 169-174, XP055162434, DE ISSN: 0931-2439, DOI: 10.1111/j.1439-0396.2007.00688.x
- CAMPBELL J M ET AL: "Selected fructooligosaccharide (1-kestose, nystose, and 1-beta-fructofuranosylnystose) composition of foods and feeds", JOURNAL OF AGRICULTURAL AND FOOD CHEMISTRY, AMERICAN CHEMICAL SOCIETY, US, vol. 45, 1 January 1997 (1997-01-01), pages 3076-3082, XP002323324, ISSN: 0021-8561, DOI: 10.1021/JF970087G

## Description

The present invention concerns a method of improving or enhancing growth at an adult age of an offspring from a female animal, or for improving or enhancing at an adult age muscle mass or muscle/fat ratio of an offspring from a female animal by administrating to said female animal during suckling of said offspring and/or to said offspring an effective amount of fructooligosaccharides.

### BACKGROUND OF THE INVENTION

Nutrition plays an essential role in developing microbiota and immune system of neonate piglets. Early-life nutrition may also influence gut immunity and metabolism at long-term and impact growth of pigs at later physiological stages by imprinting (*Guilloteau et al.*, *2010).* Indeed, modulating the diet of females during gestation and lactation and the diet of young animals results in later change in microbiota and epigenetic reactions in several species (*Lallès et al., 2012*).

Programming or imprinting is defined as "induction, silencing or restriction of development of a permanent somatic structure or physiological system with long term effects for function" (*McMillen and Robinson, 2005*)*.* This may cause by stimuli or disturbing factors acting during a sensitive time period (for example, time of maximal tissue growth, i.e., fetal life and/or beginning of the life). Programming or imprinting is based on the observation that environmental changes can reset the developmental pathways during critical periods of life, when the tissues still have some plasticity and are in a proliferating and differentiating phase. Metabolic imprinting is the process by which a stimulus, insult or a compound occurring or administrated during a critical period of development has a long-term effect on the physiologic and metabolic responses of the offspring. During development, mammals are exposed to two environments: the *in utero* environment and the postnatal environment. Maternal diet, body composition, and energy stores have major influences on both environments. Perinatal nutrition also has long lasting effects on energy balance regulation, influences susceptibility to developing metabolic disorders and plays a role in programming body weight set points. For example, it has been demonstrated that the states of maternal energy status and health implicated in predisposing offspring to obesity include maternal overnutrition, diabetes, and undernutrition. It has never been demonstrated that a dietary fibre would have an effect during the adulthood of an animal by an administration during a reduced period of time such as during the early stage of life of such animal.

As prebiotics, short-chain fructooligosaccharides (scFOS) are "selectively fermented ingredients that induce specific changes in the composition or in the activity of the gastrointestinal microflora that confer benefits to the host" (*Gibson et al., 2004*)*.* Supplementation with scFOS modulates gastro-intestinal microbiota in pigs by selective stimulation of growth and activity of beneficial bacteria such as Bifidobacteria and Lactobacilli (*Howard et al.,* 1999). As a result, scFOS enhances integrity of the intestinal barrier of piglets (*Xu et al., 2005*), modulates the immune response of vaccinated piglets (*Le Bourgot et al., 2013*), and increases insulin sensitivity in obese dogs (*Respondek et al.*, *2008).* Supplementation with scFOS of mothers and their young modulate the immune response of puppies (*Adogony et al., 2007*), thus suggesting that peri-partum supplementation with scFOS may modulate immune response. Supplementation with scFOS was disclosed as having an effect in improving growth during the supplementation period or just at the end of the supplementation period (*Grand et al.,* 2013; *Xu Chuanlai* et al., 2005; EP 0 133 547 (MEJI SEIKA CO); CN 102 613 409 (WUHAN HVSEN BIOTECHNOLOGY CO); CN 103 636 977 (ZHENJIANG TIANHE BIOLOG TECHNOLOGY CO), KIM EUN JIN et al;. 2011). This effect is not indicative of a long-term effect. In other words, an effect observed at an early age of an animal is not necessarily maintained at an adult age. Indeed generally the effect of an active ingredient or a compound is observed during administration or during a short period after the end of administration, but an effect observed several months or years after the end of administration of said compound is unusual. In the same manner, the prior art describes the use of several food additives during a specific part of the life of the animal in order to improve its growth during the supplementation time, notably during the growing period.It is the case for major products such as probiotics, organic acids. However, such recommendation is very costly and is thus poorly followed by the farmers. There is a need of a compound that can be administrated during a reduced period of time and that would enhance the growth of a mammal at its adult age.

GRAND E ET AL: "Effects of short-chain fructooligosaccharides on growth performance of preruminant veal calves." (Journal of dairy Science feb. 2013) constitutes prior art.

XU CHUANLAI ET AL: "Study of the application of fructooligosaccharides in piglets" (Asian-Australasian journal of animal sciences) constitutes prior art.

EP 0 133 547 A2 constitutes prior art.

### DESCRIPTION OF THE INVENTION

The inventors have demonstrated for the first time that early-life supplementation of an animal with FOS results in gains for breeders directly by stimulating growth of said animals.

The inventors have shown that there are sustainable metabolic and zootechnical modifications due to early-life supplementation with scFOS. Indeed, supplementation of newborn piglets with scFOS increased zootechnical performance at slaughter. Early-life supplementation with scFOS also modified the fat percentage, suggesting changes in energy metabolism pathways. The mechanisms underlying the positive effect of feeding scFOS to sows and piglets need to be further investigated but are probably related to the microbiota and the gastro-intestinal tract.

The invention concerns, a method of improving or enhancing growth at an adult age of an offspring from a female animal comprising the step of
- administrating an effective amount of short chain FOS (ScFOS) to i) the female animal during suckling of said offspring
- improving or enhancing growth at an adult age of said offspring.

The invention also concerns the use of ScFOS for improving or enhancing growth at an adult age of an animal by administrating ScFOS to its female genitor (or dam, or mother) during suckling of said animal.

According to the invention, the fructooligosaccharides are administered to said offspring or said early age animal between 2 and 4 weeks after weaning preferably 2 and 3 weeks after weaning.

According to the invention, the fructooligosaccharides are administered to said female animal or said dam at least during colostrum and/or lactating period, preferably fructooligosaccharides are administered to said female animal or said dam during end of gestation and/or during all lactating period.

As used herein, the term **"animals"** refers to any animal grown in the art of animal husbandry for use by humans for food, clothing and the like; such animals include meat-producing, for example, ruminants such as cattle, sheep and goats and nonruminants such as swine and poultry. Preferably, the animal is a non-human animal. Preferably the animal is a mammal. The term "meat-producing animals" refers to any animal grown in the art of animal husbandry for use of its meat as food. Typically, the animal is a monogastric animal. The term "monogastric animal" refers to any animal with one stomach, which applies to most carnivores and omnivores, with the exception of ruminants except the veal calves, indeed the term monogastric animal should be considered as including veal calves. The term **"standard animal feed"** refers to that feed that can be used in the animal husbandry field and is suitable to be fed to animals to supply part or all of the animals' nutrient requirements.

According to the invention, the terms **"adult age"** or **"adult animal"** refers to an animal which has reached sexual maturity.

The terms **"improving or enhancing growth at an adult age"** or "improving or enhancing growth of an animal at an adult age" refers to the measurement of an improvement or an enhancement of growth at an adult age between two adult animals one which has followed the treatment during early age and the other who has not. Indeed, according to the invention, although ScFOS are administrated at an early age during lactating period (for example for piglets 5 days to 21 days) or during between 2 and 6 weeks after weaning (for piglets weaning is between 21 days and 48 days), the improvement or enhancement of growth is observed at the adult age, meaning a long time after the end of administration of the ScFOS. In the case of pig, the animal is slaughtered at 190 days old (27 weeks). Consequently, the period between the end of the treatment and the observation of an improvement of growth is between 14 and 24 weeks meaning a period which is more than half of the pig lifespan. This effect is called "metabolic imprinting". It was very surprising that such metabolic imprinting effect can be induced by a prebiotic fiber. Moreover, as all prebiotic fibers modulate differently the gut microbiota, it was also surprising that ScFOS specifically modulate the gut microbiota and that such modulation lasts during the whole lifetime of the animal treated.

According to the invention, the term "early age" in the expression "early age animal" refers to a juvenile animal, meaning during suckling, weaning and post weaning period. The post weaning period is the period during which the animals receive pre-starter and starter feed (i.e. before the growing phase).

Typically, said animal notably said offspring may be a monogastric animal preferably a piglet and said effective amount of fructooligosaccharides is administrated to:
- said female animal or said female genitor during suckling of said offspring at 0 to 21 days and/or
- said offspring between 21 to 48 days or 21 to 40 days, preferably between 22 to 46.

The term **"effective amount"** refers to that amount of FOS preferably ScFOS that will, when administered to meat-producing animals, promotes the growth of, increases the growth rate of, and/or increases the weight gain and/or reduces the muscle/fat ratio of such meat-producing animals, without any significant adverse side effect, as compared to untreated animals. As appreciated in the art, **effective amounts** of FOS for use in the present invention will vary somewhat depending upon the particular species of animal or growth conditions such as temperature and type of feed, and the like. For any particular case, the exact or optimal effective amount to be administered can be determined by conventional dose titration techniques.

The term **"fructo-oligosaccharides"** "fructooligosaccharides" or "FOS" refers to beta-D-fructans with 2 to 10 fructose units, in which the fructose units are linked beta-2 ,1-glycosidic chain and at the terminal end there is a single D-glucose unit. Preferably, fructooligosaccharides are short-chain fructooligosaccharides. According to the invention, short chain fructooligosaccharides (scFOS) comprises 2 to 8, preferably 2 to 4 units of fructose (GF2, GF3 and GF4). Preferably, the scFOS according to the invention comprises between 28 and 24% preferably 32% (w/w) of GF2, between 40 and 54% preferably 47% (w/w) of GF3 and between 8 and 16% preferably 11% (w/w) GF4. ScFOS are generally produced from sucrose by an enzymatic or fermentation process (JP-A-56-154967 JP-B-59-53834 and JP 61-268190). ScFOS are notably commercialised under the trademark ACTILIGHT® or NUTRAFLORA® An analytic method to measure ScFOS content is also disclosed by prior art by Ouarné et al., 1999 by using an extraction step followed by an anion exchange liquid chromatography associated with an hydrolysis step using invertase. AOAC 999.03 and AOAC 999.08 also mentioned several method of analysis of FOS.

Preferably, the FOS are administrated in the form of pellets, or is comprised in a dietary supplement, or is directly added in the feed.

The invention further concerns a method of improving or enhancing at an adult age muscle mass of an offspring from a female animal comprising the step of
- administrating an effective amount of short chain fructooligosaccharides to i) the female animal during suckling of said offspring
- improving or enhancing at an adult age muscle mass of said offspring.

According to the invention the measurement of **"muscle mass"** or **"muscle/fat ratio"** is well known by the man skilled in the art. For example, an optical sensor may be used such as the optical sensor commercialized by Sydel, Lorient, France which is routinely used in the slaughter houses.

The invention also concerns a method for reducing husbandry period of an offspring from a non-human female animal, said offspring being a meat-producing animal, said method comprising the step of
- administrating an effective amount of short chain fructooligosaccharides to i) the female animal during suckling of said offspring
- reducing thereby husbandry period of said offspring.

The protein source may be provided in concentrate form, hydrolysate form, or isolate form, however the isolate form is particularly preferred for the reasons discussed herein.

Said animal protein may be selected among milk protein, casein protein, meat protein, egg protein, gelatin, fish protein, blood proteins (from blood meal) and mixtures thereof.

Marine/fish oils are oils that are obtained from aquatic animals, plants or organisms, either directly or indirectly, particularly from oily fish.

Suitable vegetal oils are coconut oil, soy oil, corn oil, olive oil, safflower oil, high oleic safflower oil, MCT oil (medium chain triglycerides), sunflower oil, oleic sunflower oil, palm oil, palm olein, and canola oil. A preferred fat source comprises at least one fatty acid, and more particularly, comprises at least one omega-3 fatty acid.

Typically such carbohydrate may be hydrolyzed, intact, naturally and/or chemically modified cornstarch, maltodextrin, maltose, glucose polymers, sucrose, corn syrup solids, rice or potato derived carbohydrate, glucose, fructose, lactose, and oligosaccharides such as fructooligosaccharides (FOS) and galacto-oligosaccharides (GOS), insulin, polydextrose, resistant starches, dextrin, and gums (i.e., Arabic), and combinations thereof. Advantageously, said carbohydrates may be in the form of flour.

The present invention will be further illustrated by the additional description and drawings which follow, which refer to examples illustrating

It should be understood however that these examples are given only by way of illustration of the invention and do not constitute in anyway a limitation thereof.

### EXAMPLES

### EXAMPLE 1: Materials and Methods

The trial was conducted from September 2012 to May 2013. According to the current legislation in France, where the whole study was performed, it will only be mandatory as from January 1st, 2013 to obtain approval from the ethical committee to conduct animal testing, thus it was not applicable when the study was performed. However, the present experiment was conducted under the direction of veterinary Bernard Sansot, who is allowed to manipulate farm animals under the permission 5509 at Faculty of Medicine of Toulouse (France).

### Animals and experimental design

The experiment was conducted in a commercial farm in France with 129 primiparous and multiparous sows (Large White × Landrace) in two replicates. The sanitary condition of the farm and zootechnical performances were typical for France. A split-plot experimental design was used with sow diet effect in the main plots and differing piglet diet effect in the subplot.

Sows at five days before parturition were allocated to one of two diets on the basis of parity at the beginning of the experiment. The two diets were a control diet (CTRL) delivered to 64 sows and a diet supplemented with scFOS (scFOS) delivered to 65 sows. The sows were in a group-housing room during gestation and moved to the farrowing room after 109±1 days of gestation to start the experiment. They were kept in individual farrowing crates (length: 2.7 m; width: 1.7 m). During the first 48 h *post-partum*, the original litter was kept with the sow. Beyond 48 h; litters were standardized to 12 piglets by cross-fostering within diet groups. Ambient temperature in the farrowing room was maintained between 22 and 25°C.

Throughout the lactation period, the piglets had no access to creep feed. They were weaned at the age of 20 days. After weaning, half of group of piglets were out of the experiment and left the farm. In the other part, all piglets remained in the same treatment group defined by their dam and were then subdivided into two groups to receive either the scFOS diet or the CTRL diet from day 21 to day 45.

The piglets stayed in a group housing room from post-weaning to slaughter. During the post-weaning phase, they were housed in pens (one female and one male as a replication) that could house up to 32 piglets. In growing and finishing phases, the piglets were housed in pens that could house up to 16 piglets. Ambient temperature in the post-weaning phase was maintained between 25 and 29°C. The pigs were fed until reaching 120 kg of BW and were then slaughtered in accordance with the current European regulations (EU regulations 1/2005 and 1099/2009). Finally, 769 pigs in 28 pens in pre-starter period were followed from weaning to slaughtering.

### Diets

All diets were formulated according to nutrient and energy requirements (Tables 1 and 2). During gestation, all the sows received two daily meals at 7:30AM and 2:00PM. They were fed on average 3.0 kg (from d 1 to 36), 2.5 kg (from d 37 to 80), and 3.5 kg (from d 81 to 109) daily of a conventional diet for pregnant sows. When they entered into the farrowing room and until weaning at d 20 after farrowing, they were fed *ad libitum* daily of a conventional diet for lactating sows (Table 1). From entering to the farrowing room until the end of lactation, the conventional diets of the sows were supplemented with either 10 g/d maltodextrins (CTRL; Tereos Syral, Marckolsheim, France), which are digestible chains of glucose molecules linked by α1-4 bonds, or 10 g/d scFOS (Profeed P95, Tereos Syral, Marckolsheim, France; 95% scFOS with molecular chain length from 3 to 5), non digestible chains of glucose and fructose linked by β (2-1) bonds.

**Table 1 Chemical analysis of the experimental diets fed by sows during gestation and lactation.**

| Item | Gestation diet | Lactation diet |
|---|---|---|
| Chemical Analysis, % as DM | | |
| CP | 15.9 | 19.8 |
| Fat | 3.0 | 4.8 |
| Crude fiber | 6.8 | 3.5 |
| Ash | 6.5 | 8.0 |
| DM, % as fed | 87.3 | 87.4 |
| DE, MJ/kg of DM | 15.0 | 16.4 |
| Lys | 0.70 | 1.16 |
| Daily allowance, kg | d 1 to d 36: 3.0 | *Ad libitum* |
| | d 37 to d 80: 2.5 | |
| | d 81 to d 112: 3.5 | |

Five conventional diets were formulated for piglets: a pre-starter diet from weaning to day 40, a starter diet from day 41 to 64; a pre-growing diet from day 65 to day 103; a growing diet from day 104 to 148, and a finishing diet until slaughter (Table 2). Fresh water was always freely available to the sows and piglets during the experimental period. After weaning and from day 21 to 40 (i.e, for the pre-starter period), the piglets of each litter were divided into 2 groups. They were fed with the conventional diet supplemented with either 1.2 g/d maltodextrins or 1.2 g/d scFOS. Maltodextrins and scFOS were directly incorporated in the diets of the sows and piglets.

**Table 2 Composition of the experimental diets formulated for piglets and growing-finishing pigs.**

| Period | Pre-starter | Starter | Pre-growing | Growing | Finishing |
|---|---|---|---|---|---|
| Age of pigs, day | 20-40 | 41-64 | 65-103 | 104-148 | 148-Slaughter |
| DM, % as fed | 88.7 | 86.9 | 87.0 | 87.2 | 86.9 |
| Chemical Analysis, % as | | | | | |
| DM | | | | | |
| CP | 21.0 | 21.4 | 20.9 | 19.8 | 18.8 |
| Fat | 7.4 | 2.0 | 2.7 | 3.0 | 3.6 |
| Crude fiber | 4.0 | 4.2 | 4.5 | 5.6 | 4.4 |
| Ash | 8.0 | 7.5 | 6.5 | 6.1 | 5.9 |
| DE, MJ/kg of DM | 16.7 | 15.2 | 15.3 | 15.3 | 15.6 |
| NE, MJ/kg of DM | 11.8 | 10.7 | 10.8 | 10.9 | 11.2 |
| Lys | 1.59 | 1.40 | 1.20 | 1.11 | 0.94 |
| Daily allowance, kg | 0.38 | 0.92 | 1.75 | 2.60 | 2.45 |

Finally, two groups of sows were formed according their diets: CTRL and scFOS groups. Four groups of piglets were distinguished: a CTRL/CTRL group in which the sows were fed with control diet and the piglets with control diet; a CTRL/scFOS group in which the sows were fed with control diet and the piglets with scFOS diet; a scFOS/CTRL group in which the sows were fed with scFOS diet and the piglets with control diet; a scFOS/scFOS group in which both the sows and piglets received scFOS diet.

### Samples and measures

**Sows.** The overall feed intake of the sows was recorded. Backfat thickness of each sow was ultrasonically measured (RENCO leanmeater, Minneapolis, USA) at farrowing and weaning of the piglets. Farrowing was assisted and the duration was registered.

**Piglets.** The number of piglets and the mortality rate were recorded at birth, at 48 h, and at the age of 21 days. Average Body Weight (BW) of each litter was measured at birth, and at weaning. The pigs were slaughtered when they reached 120 kg BW. Ages at slaughter and carcass weight were individually recorded. The percentages of fat and muscle per carcass were also individually determined by the optical sensor (Sydel, Lorient, France) used in the slaughter house.

### Statistical analysis

The observations on the sows, including feed intake, backfat thickness, litter characteristics at birth and at weaning, and farrowing duration were analyzed by ANOVA using the GLM procedure (SAS Inst. Inc., NC) with sow as the experimental unit. The model included the diet effects, parity and replicate, and the interaction between diet and parity and between diet and replicate.

5 classes of parity was made: classe 1: parity 1, n = 13 sows in CTRL group and 15 sows in scFOS group; classe 2: parity 2, n = 14 sows per dietary treatment; classe 3: parity 3, n = 11 sows in CTRL group and 13 sows in scFOS group; classe 4: parity 4, n = 13 sows per dietary treatment; classe 5: parity 5 and more, n = 11 sows in CTRL group and 12 sows in scFOS group).

When an interaction between parity and diet was significant, this was specified in the text. Data were presented as means ± Standard Deviation (SD).
Data on fattening pigs at slaughtering were analyzed by ANOVA using the GLM procedure (SAS Inst. Inc., NC) with pen as the experimental unit in a completely randomized design. The residual error was used to test the main effect of dietary treatments. The model included the effects of the diet of the sows, the diet of the piglets, replicate, and the interaction between diet of sows and diet of piglets. Means were compared using orthogonal polynomials (Gill, 1978). Data were presented as means ± SD.

### Results

### Characteristics of piglets born

The diets did not alter the total number of born piglets (15.2±2.89), the number of piglets born alive (14.4±1.79), mean BW of the litter at birth (18.6±1.05 kg), and the mortality rate of the piglets in the first 48 h (15.6±3.05%). After standardization, litter size and average BW were similar among diets (Table 3).
Litter sizes were not standardized*

**Table 3 Characteristics of newborn piglets from sows fed a control diet (CTRL) or a diet supplemented with short-chain fructooligosaccharides (scFOS) from 109 d of gestation.**

| | Diet | | | p-value |
|---|---|---|---|---|
| Item | CTRL | scFOS | SD | scFOS |
| Number of sows | 64 | 65 | | |
| Average litter size¹ at birth (born) | 14.9 | 15.4 | 2.89 | 0.362 |
| Average litter size¹ at birth (born alive) | 14.2 | 14.6 | 1.79 | 0.550 |
| % of death between birth and 48h | 15.8 | 15.3 | 3.05 | 0.803 |
| Mean litter BW at birth, kg | 18.5 | 18.7 | 1.05 | 0.776 |
| Average litter size after standardization | 12.4 | 12.7 | 2.04 | 0.154 |
| Average BW of litter after standardization, kg | 16.3 | 16.4 | 0.667 | 0.799 |

### Reproductive performance of sows

The diets did not influence body temperature 24 h after farrowing, but supplementation of the sows with scFOS decreased farrowing duration (p = 0.012, Table 4).

The diets did not affect feed intake of the sows from d 109 of gestation to weaning, and backfat thickness at farrowing. Supplementation with scFOS tended to increase backfat thickness at weaning (+0.8 mm, p = 0.091; Table 4).

**Table 4 Farrowing duration and characteristics of sows at farrowing and weaning when they fed a control diet (CTRL) or a diet supplemented with short chain fructooligosaccharides (scFOS) from 109 d of gestation until weaning, at day 20.**

| | Diet | | | p-value |
|---|---|---|---|---|
| Item | CTRL | scFOS | SD | scFOS |
| Number of sows | 64 | 65 | | |
| Farrowing duration, h. | 3.23 | 2.59 | 1.18 | 0.012 |
| Temperature 24h after farrowing, °C | 38.6 | 38.6 | 0.27 | 0.592 |
| Feed Intake, kg/day | 7.4 | 7.3 | 0.34 | 0.518 |
| Backfat thickness at farrowing, mm | 17.9 | 18.2 | 2.58 | 0.357 |
| Backfat thickness at weaning, mm | 14.1 | 14.9 | 4.40 | 0.091 |

### Growth performance of suckling piglets

The diets did not modify the total number of weaned piglets (11.3±0.06; Table 5), and piglet mortality rate from 48 h after birth to weaning. At the age of 21 days, BW and Average Daily Gain (ADG) of piglets were unaffected by maternal supplementation with scFOS (Table 5).

**Table 5 Growth performance of suckling piglets of sows fed a control diet (CTRL) or a diet supplemented with short chain Fructooligosaccharides (scFOS) from 109 d of gestation until weaning, at day 20.**

| | Diet | | | p-value |
|---|---|---|---|---|
| Item | CTRL | scFOS | SD | scFOs |
| Number of sows | 64 | 65 | | |
| Number of weaned-piglets | 11.3 | 11.3 | 0.06 | 0.965 |
| % of deaths between 48h and weaning | 7.08 | 5.48 | 8.99 | 0.193 |
| ADG* per piglet, g | 260 | 269 | 46.1 | 0.211 |
| Piglet BW* at weaning, kg | 6.16 | 6.29 | 0.66 | 0.365 |

| | | | | |
|---|---|---|---|---|
| *ADG: Average Daily Gain; BW: Body Weight | | | | |

### Growth performance at slaughtering

The piglets were slaughtered at a younger age when they received scFOS diet (-7.1 days, p = 0.004, Table 6) or when their dam was supplemented (- 6.3 days, p = 0.04). The shortest growing period was obtained in the scFOS/scFOS group (184.1± 5.48 days of age) and the longest in the CTRL/CTRL group (191.6± 5.48 days of age). BW at slaughter was not different among diets. The diets did not change muscle percentage in carcasses (64.3±0.32%; Table 6). Supplementation of the piglets with scFOS tended to reduce fat percentage in muscle (- 0.7%, p= 0.064).

**Table 6 Effects of sow and piglet supplementation with short-chain fructooligosaccharides (scFOS) compared to a control diet (CTRL) on carcass characteristics of pigs after slaughter.**

| | Diets | | | | | p-value | | |
|---|---|---|---|---|---|---|---|---|
| Item | CTRL/CTRL | CTRL/scFOS | scFOS/CTRL | scFOS/scFOS | RSD | scFOS sow | scFOS piglets | Sow × piglets |
| Number of pens | 7 | 7 | 7 | 7 | | | | |
| Number of piglets | 191 | 194 | 193 | 191 | | | | |
| Age at slaughter, d | 191.6^{a} | 185.0^{b} | 186.5^{b} | 184.1 ^{b} | 5.48 | 0.041 | 0.004 | 0.151 |
| Carcass weight, kg | 95.9 | 96.0 | 95.7 | 95.8 | 0.01 | 0.694 | 0.829 | 0.978 |
| Muscle, % | 63.9 | 65.1 | 63.7 | 64.6 | 0.32 | 0.754 | 0.390 | 0.914 |
| Fat, % | 16.2 | 15.9 | 16.7 | 16.0 | 0.53 | 0.218 | 0.064 | 0.464 |

### Discussion

The study was performed to evaluate the reproductive performance of sows after scFOS supplementation during the five last days of gestation and throughout the 20 day-lactation period, and to evaluate the growth performance of the pigs subjected to an early-life supplementation with scFOS. The effects of scFOS on sow colostrum immune quality and on immune response of piglets subjected to a challenge were also assessed. No sanitary problems that could have disrupted the trial occurred. scFOS and control diets were well consumed by both the sows and the piglets. Thus, we assume that experimental conditions were met to achieve the aims of the study.

### Supplementation of sows with scFOS modifies reproductive performance

ScFOS supplementation reduced farrowing duration. A short farrowing duration is important for piglets' survival and for subsequent reproductive performance of sows (Friend et al., 1962). Many factors affect farrowing duration, including breed, sow age, gestation length, number of born piglets, sow body condition, and constipation state (Oliviero et al., 2010). Breed, sow age, gestation length, and number of born piglets were similar in both sow groups. No measure of constipation state has been realized in the present study, but scFOS are known to regulate intestinal transit in humans (Paineau et al., 2008). Thus, scFOS may have decreased farrowing duration by improving intestinal transit of sows.

Backfat thickness at weaning tended to be higher in sows fed scFOS compared to those fed control diet while intake was similar between the 2 groups of sows. Le Bourgot et al. (2013) observed similar effects on backfat thickness in sows fed with scFOS diet during the 4 last weeks of gestation and lactation. Late-pregnancy and lactation are specific physiological periods in which sows may become insulin-resistant, which stimulates mobilization of energy storage (Père and Etienne, 2007; Farmer and Quesnel, 2009). As a prebiotic ingredient, scFOS modulate microbiota and increase VFA production in colon of many species, including pigs (Tsukahara et al., 2003). They also enhance insulin sensitivity in both obese and healthy animals (Massimo et al., 1998; Respondek et al., 2008). The relationship between dietary scFOS supplementation, microbiota modulation, and reduction of blood insulinemia has been recently highlighted in humanized gnotobiotic diet induced obesity mice (Respondek et al., 2013). Thus, scFOS could increase insulin sensitivity of sows and by this way decrease mobilization of adipose tissue for fatty acid synthesis during lactation.

All values reported for sow colostrum composition, including fat and energy, are in agreement with biological values previously reported by Klobasa et al. (1987) and Le Dividich et al. (2004). However, energy and fat contents were slightly lower in colostrum of sows fed with scFOS than with CTRL diet, while feed intake and the number of suckling piglets were similar. This is consistent with the possible lower mobilization of adipose tissue previously mentioned in sows fed scFOS. The difference in colostrum nutritional composition did not impact the mortality rate of piglets or BW at weaning, suggesting piglets of sows fed with scFOS diet received sufficient energy quantity to ensure survival and growth.

### ScFOS supplementation results in long-term effects on growth performance

The animals were slaughtered at a younger age with sow and piglet supplementation. Similarly, Le Bourgot (INRA, Rennes, France, personal communication) observed a positive carry-over effect of scFOS supplementation of sows on BW of their piglets at the age of 77 days. Supplementation with scFOS also increased growth and feed efficiency when directly fed to veal calves (Grand et al., 2013) or piglets (Howard et al., 1999; Xu et al., 2005). However, these previous studies evaluated the effects of scFOS on growth performance during supplementation, while the present study highlighted a positive carry-over effect of early-life nutrition on later growth performance.

In addition to changes in their growth curve, early-life supplementation of piglets with scFOS also modified carcass characteristics. Indeed, supplementation during the pre-starter period tended to decrease fat percentage in muscles measured on carcass. Numerous studies have been designed to evaluate impacts of early nutrition on piglet growth performance but they mainly focused on protein levels or on feed restriction (Sarr et al., 2010; Sarr et al., 2011). To our knowledge, no data is available on the effects of prebiotics given to piglets on their later growth performance.

ScFOS may affect energy metabolism pathways when directly fed to piglets or to their lactating mothers. ScFOS supplementation of sows may modify energy metabolism of piglets, probably through a modification of milk composition (Le Bourgot et al., 2012). Long-term effects of scFOS supplementation of sows and piglets are also undoubtedly related to a modulation of intestinal microbiota. Evidence for the involvement of underlying epigenetic modifications in long-term studies emerges with regard to the gastrointestinal tract. More precisely, microbiota is involved in modulating the host epigenome at the gut level (Hinnebusch et al., 2003; Takahashi et al., 2011). Such results, in addition to the well-known prebiotic properties of scFOS, lead to the idea that scFOS may modulate early bacterial colonization of piglets and by this way, modulate metabolic responses at later physiological stages.

## Claims

1. A method of improving or enhancing growth at an adult age of an offspring from a female animal comprising the step of
- administrating an effective amount of short chain fructooligosaccharides comprising 2 to 8 units of fructose to the female animal during suckling of said offspring
- improving or enhancing thereby growth at an adult age of said offspring.

2. The method according to claim **1**, wherein the fructooligosaccharides are administered to said offspring between 2 and 4 weeks after weaning, preferably 2 and 3 weeks after weaning.

3. The method according to claim **1** or claim **2**, wherein the fructooligosaccharides are administered to said female animal at least during colostrum and/or lactating period, preferably fructooligosaccharides are administered to said female animal during end of gestation and/or during all suckling period.

4. The method according to any one of claims **1** to **3**, wherein said offspring is a non-human animal, preferably a mammal.

5. The method according to any one of claims **1** to **4**, wherein said offspring is a monogastric animal, preferably a piglet, and wherein said effective amount of fructooligosaccharides is administrated to:
- said female animal during suckling of said offspring at 0 to 21 days and/or
- said offspring between 21 to 48 days.

6. A method of improving or enhancing at an adult age muscle mass or muscle/fat ratio of an offspring from a female animal comprising the step of
- administrating an effective amount of short chain fructooligosaccharides comprising 2 to 8 units of fructose to the female animal during suckling of said offspring
- improving or enhancing thereby at an adult age muscle mass or muscle/fat ratio of said offspring.

7. A method for reducing husbandry period of an offspring from a non-human female animal, said offspring being a meat-producing animal, said method comprising the step of
- administrating an effective amount of short chain fructooligosaccharides comprising 2 to 8 units of fructose to the female animal during suckling of said offspring
- reducing thereby husbandry period of said offspring.

8. Use of a short chain fructooligosaccharide comprising 2 to 8 units of fructose for improving or enhancing growth at an adult age of an animal by administrating said fructooligosaccharide to its dam during suckling of said animal.

## Patentansprüche

1. Ein Verfahren zur Verbesserung oder Erweiterung des Wachstums in einem Erwachsenenalter eines Nachkommen von einem weiblichen Tier, umfassend den Schritt des
- Verabreichens einer wirksamen Menge an kurzkettigen Fructo-Oligosacchariden, 2 bis 8 Einheiten an Fructose umfassend, an das weibliche Tier während des Säugens des Nachkommen
- Verbesserns oder Erweiterns dadurch des Wachstums in einem Erwachsenenalter des Nachkommen.

2. Das Verfahren nach Anspruch **1,** wobei die Fructo-Oligosaccharide dem Nachkommen zwischen 2 und 4 Wochen nach dem Absetzen, vorzugsweise 2 und 3 Wochen nach dem Absetzen verabreicht werden.

3. Das Verfahren nach Anspruch **1** oder Anspruch **2,** wobei die Fructo-Oligosaccharide dem weiblichen Tier mindestens während des Erstmilch- und/oder Milch absondernden Zeitraums verabreicht werden, wobei die Fructo-Oligosaccharide dem weiblichen Tier vorzugsweise am Ende der Tragezeit und/oder während des gesamten Säugezeitraums verabreicht werden.

4. Das Verfahren nach einem der Ansprüche **1** bis **3,** wobei der Nachkomme ein nicht-menschliches Tier, vorzugsweise ein Säugetier ist.

5. Das Verfahren nach einem der Ansprüche **1** bis **4,** wobei der Nachkomme ein monogastrisches Tier, vorzugsweise ein Ferkel ist, und wobei die wirksame Menge an Fructo-Oligosacchariden verabreicht wird an
- das weibliche Tier während des Säugens des Nachkommen an 0 bis 21 Tagen und/oder
- den Nachkommen zwischen 21 bis 48 Tagen.

6. Ein Verfahren zur Verbesserung oder Erweiterung einer Muskelmasse im Erwachsenenalter oder eines Muskel-/Fettverhältnisses eines Nachkommen eines weiblichen Tieres, umfassend den Schritt des
- Verabreichens einer wirksamen Menge an kurzkettigen Fructo-Oligosacchariden, 2 bis 8 Einheiten an Fructose umfassend, an das weibliche Tier während des Säugens des Nachkommen
- Verbesserns oder Erweiterns dadurch einer Muskelmasse oder eines Muskel-/Fettverhältnisses im Erwachsenenalter des Nachkommen.

7. Ein Verfahren zur Verringerung des Haltungszeitraums eines Nachkommen eines nicht-menschlichen weiblichen Tieres, wobei der Nachkomme ein fleischerzeugendes Tier ist, wobei das Verfahren die Schritte umfasst des
- Verabreichens einer wirksamen Menge an kurzkettigen Fructo-Oligosacchariden, 2 bis 8 Einheiten an Fructose umfassend, an das weibliche Tier während des Säugens des Nachkommen
- Verringerns dadurch des Haltungszeitraums des Nachkommen.

8. Verwendung eines kurzkettigen Fructo-Oligosaccharids, 2 bis 8 Einheiten an Fructose umfassend, zur Verbesserung oder Erweiterung des Wachstums in einem Erwachsenenalter eines Tieres durch Verabreichung des Fructo-Oligosaccharids an dessen Mutter während des Säugens des Tieres.

## Revendications

1. Un procédé pour améliorer ou renforcer la croissance à l'âge adulte d'une progéniture d'un animal femelle, comprenant l'étape
- d'administrer une quantité efficace de fructooligosaccharides à chaîne courte comprenant 2 à 8 unités de fructose à l'animal femelle pendant l'allaitement de ladite progéniture
- améliorant ou renforçant ainsi la croissance à l'âge adulte de ladite progéniture.

2. Le procédé selon la revendication **1,** dans lequel les fructooligosaccharides sont administrés à ladite progéniture entre 2 et 4 semaines après le sevrage, de préférence 2 et 3 semaines après le sevrage.

3. Le procédé selon la revendication **1** ou la revendication **2,** dans lequel les fructooligosaccharides sont administrés audit animal femelle au moins pendant la période de colostrum et/ou de lactation, de préférence les fructooligosaccharides sont administrés audit animal femelle pendant la fin de la gestation et/ou pendant toute la période d'allaitement.

4. Le procédé selon l'une quelconque des revendications **1** à **3,** dans lequel ladite progéniture est un animal non-humain, de préférence un mammifère.

5. Le procédé selon l'une quelconque des revendications **1** à **4,** dans lequel ladite progéniture est un animal monogastrique, de préférence un porcelet, et dans lequel ladite quantité efficace de fructooligosaccharides est administrée à :
- ledit animal femelle pendant l'allaitement de ladite progéniture entre 0 et 21 jours et/ou
- ladite progéniture entre 21 et 48 jours.

6. Un procédé pour améliorer ou renforcer à l'âge adulte la masse musculaire ou le rapport muscle/graisse d'une progéniture d'un animal femelle, comprenant l'étape
- d'administrer une quantité efficace de fructooligosaccharides à chaîne courte comprenant 2 à 8 unités de fructose à l'animal femelle pendant l'allaitement de ladite progéniture
- améliorant ou renforçant ainsi à l'âge adulte la masse musculaire ou le rapport muscle/graisse de ladite progéniture.

7. Un procédé pour réduire la période d'élevage d'une progéniture d'un animal femelle non-humain, ladite progéniture étant un animal producteur de viande, ledit procédé comprenant l'étape
- d'administrer une quantité efficace de fructooligosaccharides à chaîne courte comprenant 2 à 8 unités de fructose à l'animal femelle pendant l'allaitement de ladite progéniture
- réduisant ainsi la période d'élevage de ladite progéniture.

8. Utilisation d'un fructooligosaccharide à chaîne courte comprenant 2 à 8 unités de fructose pour améliorer ou renforcer la croissance à un âge adulte d'un animal en administrant ledit fructooligosaccharide à sa mère pendant l'allaitement dudit animal.
